# EUROPEAN PATENT APPLICATION

(11) **EP 2 523 135 A1**
(43) Date of publication of application: **14.11.2012**
(21) Application number: 12159067.3
(22) Date of filing: 12.03.2012
(51) Int. Cl.: G06F 19/00

(54) **Medical simulation system**

(30) Priority: 11.05.2011 US 201161484724 P; 11.05.2011 GB 201107790
(71) Applicant: Laerdal Medical AS, 4002 Stavanger (NO)
(72) Inventor: Eikefjord, Arild Jarle, 4002 Stavanger (NO)
(74) Representative: Sonnenberg, Fred

(57) **Abstract**

A computerized educational apparatus (10) and method for simulating medical events is disclosed. The apparatus (10) comprises a patient simulator (20) having a plurality of sensors (225) and a plurality of actuators (220); a control device (40) located remotely from the patient simulator (20); a base unit (30) in data contact with the manikin (20) and the control device (40); and one or more medical parameter measurement devices (21, 22, 70) in data contact with the base unit (30).

## Description

### Field of the Invention

The field of the invention relates to a computerised educational apparatus, a control device, a method for simulating patient care and a patient simulator.

### Background of the invention

A manikin - also called a patient simulator - is a life-sized anatomical human model used as a teaching aid in medical education for training doctors, nurses, paramedics as well as other learners in, for example, emergency care and resuscitation of humans. A number of companies produce manikins. For example, Laerdal Medical AS, Stavanger, Norway, have produced manikins in various forms since the 1960s. Generally manikins are three-dimensional models of all or part of a human being and are intended to be as realistic as possible in order to provide the learners with a realistic situation. The manikin can be used to instruct learners using a so-called "training scenario". The training scenarios are designed to be realistic simulations of medical emergencies that might occur in real-life. An instructor can institute one or more of the training scenarios and view how the learner responds to the implemented training scenario.

US Patent No. 5,853,292 (Eggert et al, assigned to Gaumard Scientific) teaches an interactive, computerized education system for teaching patient care to a learner. The system includes an interactive computer program for use with a patient simulator, such as a manikin, and virtual instruments for performing simulated patient care activity under the direction of the interactive computer program. The interactive computer program displays a selection of modules, i. e. training scenarios, to assist the learner in learning patient care protocols. The modules are selectable by the user for providing different interactive training sessions involving the patient care protocols. The virtual instruments are used with the patient simulator in performing the patient care activity. The virtual instruments cooperate with sensors that interface with the computer program for providing feedback to the interactive computer program regarding the activity and confirming proposed placement and use of the virtual instruments on the patient simulator.

The Computer Interface Module (CIM) of US `292 is connected to a computer via a line. The CIM further comprises a number of ports to which virtual instruments and sensors are connected The processor circuit of the CIM processes received input signals to provide feedback information to the computer pertaining to activity performed by a user on the manikin. The information is used by the computer program to provide interactive training sessions. The task of the processor circuit is to simply translate sensor input signals into data send to the computer or to simply translate data received from the computer into signals to actuate the actuators.

US '292 does not disclose that the CIM provides intelligent functions which would change the signals sent to the actuators, for example over time. The computer program effects all changes and the CIM merely translate this changes, without applying any adjustments to the data received from the computer.

Other patents and patent applications are known which describe various computer architectures for interactive education systems for teaching page and care. For example, US Patent No. US 7,277,874 (American Board of Family Medson, Lexington, Kentucky) describes a method and system for patient generation and evolution for a computer-based testing system and/or expert system. US patent No. US 7,653,556 (American Board of Family Medson, Lexington, Kentucky) discloses a computer-implemented simulation and evaluation method which simulates interventions to a patient by a learner and is then able to evaluate the interventions responsive to predetermined criteria and interventions. The method taught in the US `556 patent includes defining a test area to evaluate the learner to at least one of predetermined criteria and a learner profile.

US patent application publication No. US 2010/0304347 (Gaumard Scientific) discloses an interactive education system for teaching patient care to a user. The system comprises a patient simulator (manikin), a virtual instrument for use with the patient simulator and means for sensing interactions between the virtual instrument and the patient simulator.

International Patent Application No WO 2009/088308 (Laerdal Medical) teaches a method, system and computer program product for providing a simulation, such as for teaching patient care, that provides advanced notification of simulation events. TheWO '308 patent application can be used in computerised educational apparatus, such as a patient simulator.

### Summary of the invention

A computerized educational apparatus for simulating medical events is disclosed. The apparatus comprises, at least, one patient simulator (also termed manikin) having a plurality of sensors and a plurality of actuators, a control device located remotely from the patient simulator, a base unit in data contact with the manikin and the control device, as well as and one or more medical parameter measurement devices in data contact with the base unit. The control device comprises a state parameter memory which is used to store a plurality of medical state parameters and which is coupled to said control processor. The medical state parameters are representative of a medical state of at least one the patient simulators. The use of the remotely located control device enables the patent simulator to be controlled by an instructor observing a learner and to react in a short time. The control device can also enable downloading of a plurality of learning scenarios to the patient simulator. The medical state parameters used in the learning scenarios can be changed by the instructor using the remotely located control device in order to change the simulation of the medical event represented by the learning scenario.

The remotely located control device is adapted to communicate with the base unit of the patient simulator for the transfer of medical state parameter values that are representative of the medical state parameters. The base unit is adapted to store changes received from the control device in a base unit memory and is further adapted to calculate in a base unit processor those changes that need to be sent to the patient simulator. The plurality of medical state parameters are representative of a medical state of the patient simulator and can be changed dynamically by the instructor.

In one aspect of the disclosure the control device further comprises an accelerometer connected to the graphics processor. The accelerometer detects the position of the portable control device and cooperates with the graphics processor to adapt a view on the display.

In one aspect of the disclosure, the base unit processor is able to take into account time change parameters received from the remotely located control device and then able to calculate over time changes in the learning scenario which are sent to the patient simulator. The instructor does not actively change the parameters that leads to a more reliable learning scenario.

A patient simulator is also disclosed. The patient simulator has at least one loudspeaker, at least one or more body parts, at least one or more actuators adapted to move at least one or more of the body parts, at least one or more sensors adapted to sense manipulation of at least one of the one or more body parts, and at least one or more embedded controllers adapted to transceive data signals from a base unit and connected to at least one of the one or more sensors or the one or more actuators. The patient simulator is adapted to be in data contact with the remotely located base unit.

A method for simulating patient care in a patient simulator is also disclosed. The method comprises setting one or more medical state parameters, detecting parameter changes in at least one of the one ore more medical state parameters in a control device, transmitting said parameter changes from the control device to a base unit, storing the parameter changes and then calculating in the base unit those changes that need to be adjusted in the patient simulator. The changes are used to adjust at least one of a plurality of actuators or a plurality of sensors in the patient simulator.

Finally, a method for changing medical state parameter values is also disclosed and comprises selecting in a control device a medical state parameter from a plurality of medical state parameters, displaying a first medical state parameter value of the selected medical state parameter in the control device, detecting movement of an object from a first position corresponding to the first medical state parameter value to a second position corresponding to a second medical state parameter value of the selected medical state parameter, and storing the second medical state parameter value.

### Description of the figures

Fig. 1 shows an overview of the computerized educational apparatus;

Figs. 2a - 2c show example of manikins;

Fig. 3 shows an example of the audio connections between base unit and manikin;

Figs. 4a to 4e show an examples of a display on a control unit.

Fig. 5 shows a flow diagram of a testing method.

### Detailed description of the invention

The invention will now be described on the basis of the drawings. It will be understood that the embodiments and aspects of the invention described herein are only examples and do not limit the protective scope of the claims in any way. The invention is defined by the claims and their equivalents. It will be understood that features of one aspect or embodiment of the invention can be combined with a feature of a different aspect or aspects and/or embodiments of the invention.

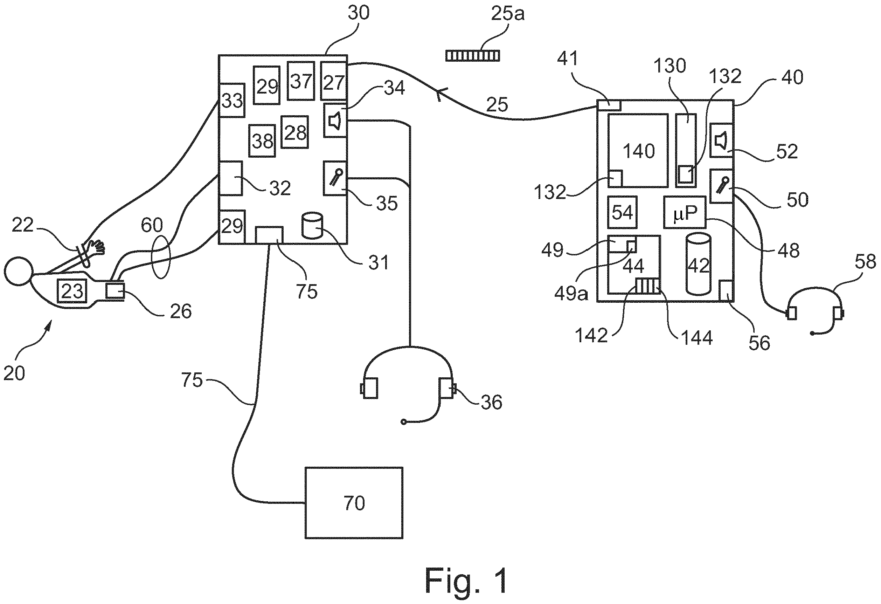

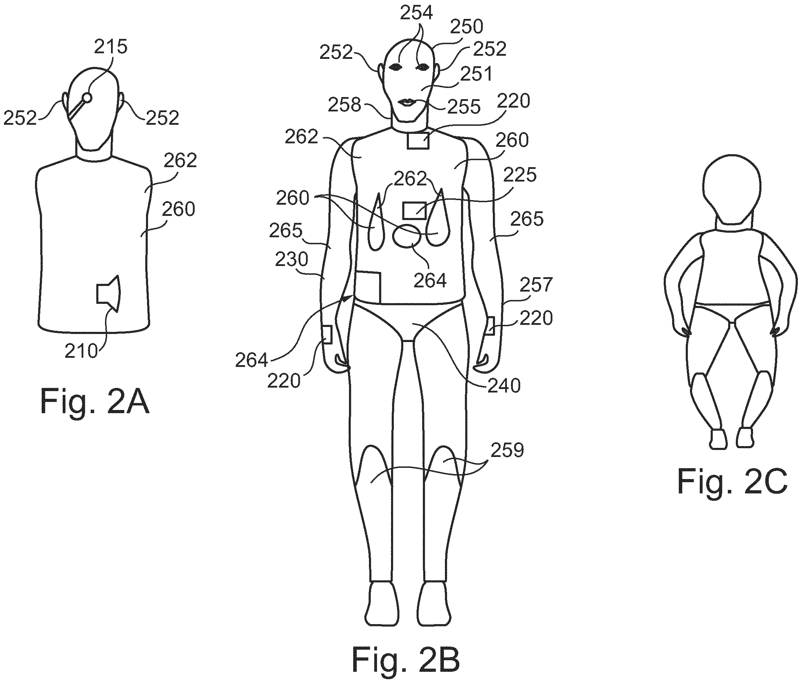

Fig. 1 shows an overview of a computerized educational apparatus 10 comprising a patient simulator or manikin 20, a base unit 30 and a control unit 40. The base unit 30 may reside inside the manikin 20 or may be a separate unit located near the manikin 20 or more remotely. The manikin 20 in this aspect of the invention is a life-sized anatomical human model. More generally the manikin 20 is a three-dimensional model of all or part of a human being and are intended to be as realistic as possible in order to provide the learners with a realistic situation termed a training scenario. The manikin 20 shown in Fig. 1 is the size of an adult. The manikin 20 could, of course, be a baby as shown in Fig. 2c, an infant, a child, a pregnant woman, or a torso (as shown in Fig 2b) and are constructed depending on the needs of the learners and/or the training scenarios.

Figs. 2a - 2c show example of the manikin 20 used in the computerised training system of the disclosure. The manikin 20 includes the typical features of a human patient, such as a head 250, a face 251, two ears 252, two eyes 254, a mouth 255, a torso 260, a chest 262, a pair of lungs 262, a heart 264, knees 254, wrists 257, neck 258, thighs 261 or elbows 265. It will be appreciated that the manikin 20 may miss one or more of the features, depending on the training scenarios. The manikin 20 is covered with a skin 230 and may or may not have clothing 240.

The manikin 20 further includes one or more loudspeakers 210 to make typical patient sounds. The typical patient sounds are generated in the base unit 30 or the control unit 40, as will be explained later. Preferably the manikin 20 includes more than one loudspeaker 210 placed at different positions. So, for example there may be one loudspeaker 210 placed near to a mouth 255 of the manikin 20 to generate mouth-type sounds (as well as speaking) and another one of the loud speakers 210 placed near the heart 264 to generate heart sounds. The manikin 20 can have one or more microphones 215 to enable the learner to "communicate" with the manikin 20 (and in fact with the instructor). Such microphones 215 could be placed for example near to the ears 252 to resemble real-life communication.

The manikin 20 also has typically a manikin identification 270 that indicates the type of manikin 20. The manikin identification 270 could be either "hard-wired" into a memory chip or by using a combination of switches in on and off positions. The manikin identification 270 is interrogated by the base unit 30 on connection to the manikin 20 to enable the correct data signals using a correct protocol to be sent to the manikin 20.

The manikin 20 further includes a plurality of actuators 220 which can be used to mimic various functions within the manikin 20, such as but not limited to pulse measurements and movements of the neck 258. The actuators 220 include, but are not limited to, valves and solenoids. The manikin 20 also includes a plurality of embedded processors 26 which can be used to control the actuators 220.

The manikin 20 further includes a plurality of sensors 225 which can be used to respond to actions of the learner on the manikin 20. For example, the sensors 225 could measure an attempt by the learner to defibrillate the patient, to palpate a pulse, or to provide ventilation. The sensors 225 can be pressure sensors, light sensors, or fluid sensors. The plurality of embedded processors 26 within the manikin 20 can be used to connect and to the transmit data signals from the sensors 225 to the base unit 30. A simulated thermometer 21 or other simulated instruments, such as but not limited to pulse oximeter, glucose meter, capnographcapograph for measuring the concentration of carbon dioxide in the air passage or other patient monitoring device may also be connected to the manikin 20.

The base unit 30 in Fig. 1 is connected to the manikin 20 by control and data leads 60. It will be appreciated that the base unit 30 could be connected to the manikin 20 by a wireless, Bluetooth or similar connection. The base unit 30 may also have a battery or other means of power transfer. The control unit 40 is connected to the base unit 30 by a data link 25 that is a wireless connection, e.g. using a wireless LAN or a Bluetooth connection. It will be appreciated, however, that the connection between the control unit 40 and the base unit 30 could be by a cable using, for example, an Ethernet protocol for the transmission of data. The base unit 30 can be connected to a personal computer or computer network through an Ethernet cable, a USB connection or via a wireless LAN. The connection of the control unit 40 to a personal computer or the computer network enables the uploading of data from an implemented training scenario and a downloading of customised training scenarios.

The control unit 40 has a network client 41 (or network interface) battery 42, a memory 44, a display 46 and a processor 48. The network client 41 communicates to the base unit 30 through the data link 25. The display 46 is a touchscreen colour display, for example made of LCDs. It will be appreciated that other types of the display 46 are possible. The use of the touchscreen colour display 46 enables the instructor using the control unit 40 to merely use either his or her fingers or a stylus to operate the control unit 40 and thus change parameters relating to a training scenario sent to the manikin 20, as will be explained later. It will be appreciated that it is possible to connect a keyboard, joystick, mouse, or similar input devices to the control unit 40 if and when required. It will be appreciated that it is possible to also generate on the display 46 a "virtual" keyboard that can be operated by touching the display 46 in sectors corresponding to letters displayed on the virtual keyboard. The touchscreen is, for example, implemented as a capacitance touchscreen and covers the entire area or part of the touchscreen display 46. The touchscreen display 46 can allow for "multitouch" in which more than one finger simultaneously touches the display 46 and the touch of all of the fingers in corresponding sectors of the display 46 is recorded.

The control unit 40 may also have an audio input 50 to allow recording of notes by the instructor or communication to the learnerstrainees through the manikin 20 and an audio output (speaker 52) which can give, for example, warning sounds but also messages to the instructor. The audio input 50 and the audio output 52 can be connected to a headset 58. An accelerometer 54 is included in he control unit 40 and can detect orientation of the display 46, i. e. if the display 46 is held in a portrait or landscape view or if the control unit 40 is held upside-down or laying flat. The arrangement on the display 46 can be changed depending on the orientation of the display 46. A data interface 56, for example a USB interface, an SD card interface or CF card interface, in the control unit 40 allows the uploading of the customised training scenarios and also storage of training results data relating to the implemented training scenario. An SD card can be incorporated permanently into the control unit 40 for storage of data.

The processor 48 can be one of many types of processors produced by ARM, Intel or MIPS. The processor 48 runs an operating system, such as, but not limited to Linux operating system or Windows Mobile operating system.

It will be appreciated that the control unit 40 has an active power management system which is designed to save the life of the battery 42 by turning off a backlight of the display 46 when not the display 46 is in use and also shutting down the control unit 40 after periods of inactivity and/or depending of movement and/or orientation of the control unit 40. This timing out of the control unit 40 can be varied depending on the training scenarios and desires of the instructor. The control unit 40 will also have a charging device directly incorporated into the control unit 40 or can be connected to an external supply for re-charging the batteries 42.

The base unit 30 also has a base unit battery 31 or other power source, e. g. connection to mains supply. The base unit 30 is connected to the manikin 20 by the control and data leads 60 through a cable connector 32 which is, for example, a 60 pin female connector and includes the following signals on one or more of the pins of the connector: power to the manikin 20; audio outputs (including but not limited to vocal sounds, left lung and right lung, heart, bowel, blood pressure); pulse drive and sense signals (including but not limited to left carotid pulse and right carotid pulse, brachial pulse, radial pulse); ECG output; signals relating to a measurement of a defibrallatory shock or external pacing performed on the manikin 20, manikin identification input signal (connected to the manikin identification 264); chest compression measurement and feedback device, light; on-off signal. The base unit 30 has an air pressure input 33 that is connectable to a blood pressure cuff 22 on the manikin 20. The base unit 30 also has a base unit memory 38 that is used to store information about different functions that the base unit 30 can implement in the manikin 20. The simulated thermometer 21 or other simulated instruments are connected to the base unit 30 through a wireless link.

The base unit 30 also has a controller area network (CAN) interface which provides a link to the embedded processors 26 in the manikin 20 over the control and data leads 60.

The base unit 30 has an audio output 34 which can be connected to a loudspeaker or to a base unit headset 36 and an audio input connectable to a microphone or the base unit headset 36. The base unit 30 has a sounds generator 37 which is connected through the control and data leads 60 to speakers 23 in the manikin 20.

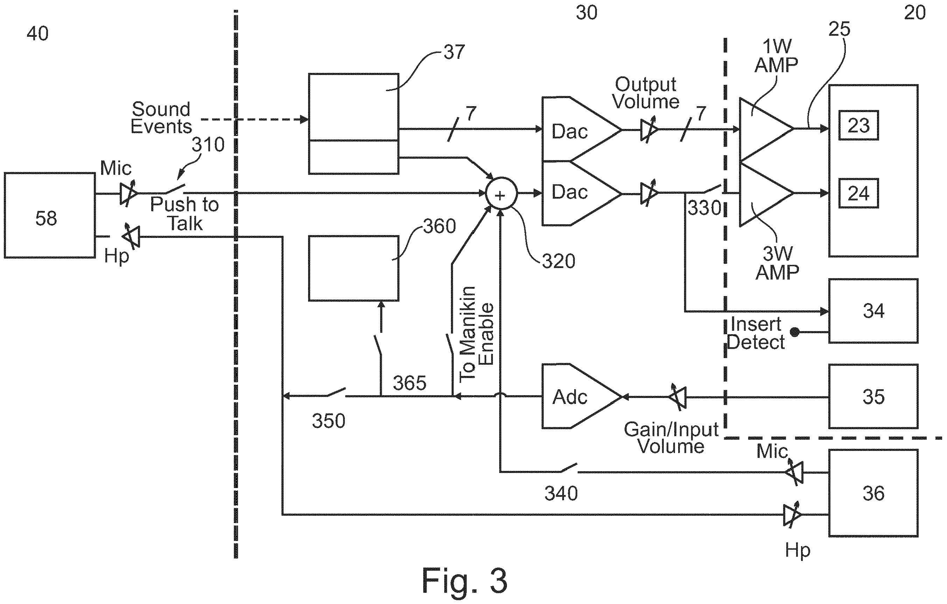

Fig. 3 shows an example of the audio connections 300 between the control device 40, the base unit 30 and the manikin 20. The same reference numerals are used on Fig. 3 to identify the same or similar elements to those present on Fig. 1. It will be seen that in the aspect of the disclosure shown in Fig. 3 the instructor can communicate with the learner through the control device headset 58 by pushing an instructor talk button 310 in the control device 40. The base unit 30 has a router 320 which routes voice signals and sound signals to the appropriate elements. The base unit 30 or the manikin 20 has a manikin vocal enable switch 330 which can be operated either locally or by the instructor to enable the learner to communicate with the manikin audio input 24 (and thus indirectly to the instructor). The learner can be wearing the base unit headset 36 and this includes a headset talk switch 340 to enable communication to be made to the instructor. It will be seen that the base unit 30 further includes a recording device 360, typically as a solid state memory, which is enabled by a memory switch 365 to enable recordings to be made of any dialogue between the instructor and the learner as well as the sounds generated in the manikin 20. It should be noted that the audio connections 300 are merely exemplary and that different connections can be made between the control device 40 and the base unit 30 as well as the manikin 20.

The base unit 30 has a base unit processor 38 which cooperates with parameters stored in the base unit memory 38 to generate one or more different training scenarios in the manikin 20. So for example, the base unit 30 can generate signals along the control and data leads 60 to enable one or more of the actuators 200 in the manikin 20 to reproduce pulses. It will be appreciated that the learner can be expected to look for a pulse in the wrist 257, the neck 258 or the thigh 261 of the manikin 20. Thus it will be expected that there are the actuators 220 in these areas to enable the learner to "feel" the pulse through the skin 230 of the manikin 20. It will also be expected that some of the sensors 225 are located in these areas to sense whether the learner has correctly attempted to sense the pulse. The skilled person will note that the pulse can be felt in different places on the manikin 20 and further ones of the actuators 220 and/or the sensors 225 can be included in, for example, the knee 259 or the chest 262 to represent the pulse. It will be noted that some of the actuators 210 may perform "multiple" functions as will be explained below.

The base unit processor 39 can generate the pulses or different arrhythmias with heartrates at many different rates. For example at resting (around 80 beats per minute), very low (less than 60 beats a minute) or extremely high (e.g. 140 beats per minute) depending on the implemented training scenarios. The base unit processor 39 can generate carotid pulses (in the neck 258), femoral pulses (in the thigh 261), brachial pulses (in the elbow 265) and radial pluses (at the wrist 257). The base unit processor 39 will ensure that the pulses generated are "compatible" with each other and will also check that the apparent blood pressure in the manikin 20 is such that the radial pulse is, for example, detectable at the wrist 257.

The base unit processor 39 can generate electro-cardiac rhythms (arrhythmias). These electro-cardiac rhythms are sent to themanikin 20 where the electro-cardiac rhythms may be detected are viewed by the learner with e.g. a heart monitor connected to the manikin 20 by EDG leads. It will be appreciated that the electro-cardiac rhythms are coordinated with the pulses generated by the base unit processor 39.

The base unit processor 39 cooperates with the sounds generator 37 to produce typical patient sounds in the manikin 20 as described above. Such typical patient sounds can include, but are not limited to, lung sounds, heart sounds, bowel sounds, vocal sounds. The sound channels are individually settable, but some sounds are related to other sounds and this relationship is either hardwired into the manikin 20 or the base unit 30 or preset in the base unit processor 39 or the sounds generator 37.

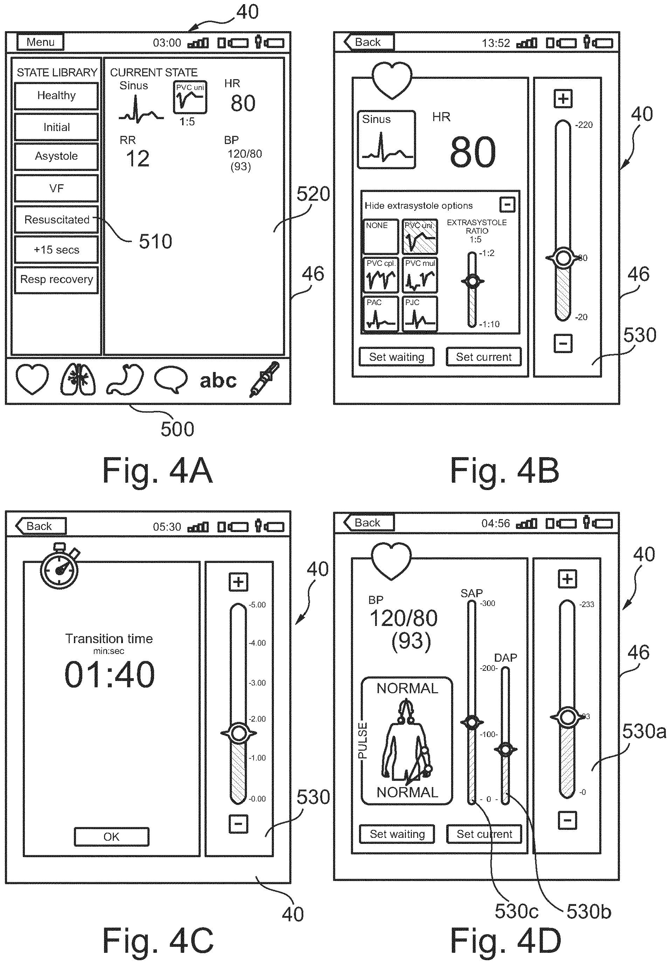

Figs. 4a - 4e show examples of the control unit 40 with the display 46 illustrated in more detail. The control unit 40 is designed to be used by the instructor and includes all of the relevant information relating to the instructions sent to the control unit 30 for training scenario implementation in the manikin 20. The design on the screen of the display 46 is governed by a graphical user interface application 132 running in the graphics processor 130. There is a plurality of screens for the display 46 which the instructor can select. The main screen is shown in Fig. 4a. The display 46 includes a selection of subscreen selection buttons 500 on the bottom with icons for selecting appropriate ones of the subscreens. It will be appreciated that the display of the subscreen selection buttons 500 in the bottom is merely illustrative and that the GUI application 132 can select a design based on orientation of the control unit 40 and/or information.

State buttons 510 are included on the right hand side and illustrate a selection of predefined patient states selected from a state library stored in a state parameter memory 142 in the control unit. The state parameter memory 142 includes "typical" states or pre-programmed changes in state that the instructor may wish to select for implementation during a training and/or evaluation session. The typical states and/or pre-programmed changes in state are stored as medical state parameters 144. The summary display area 520 shows the values of the main parameters relating to the vital functions being simulated in the manikin 20 during the training scenario. In the example shown this summary display area 520 includes heart rate (HR), respiration rate (RR) and blood pressure (BP), but it will be appreciated that the selection of the vital functions illustrated on the main screen in Fig. 4a is illustrative and can be changed.

The instructor can select any of the subscreen selection buttons 500 to move to appropriate ones of the subscreens. For example, selection of the heart icon will move to a subscreen shown in Fig. 4b in which the vital functions relating to the heart 264 are displayed. The instructor can change any one of the values of the medical state parameters relating to vital functions by selecting the appropriate area of the display 46 with a sliding bar 530. The instructor can move the sliding bar 530 using a finger or stylus to change the value of the medical state parameter represented by the sliding bar. The instructor can also specify the length of time over which the change should take place (as will be discussed below in connection with Fig. 4C).

The changing of any one of the medical state parameters is carried out by using a C++ object 49 stored in the memory 44 and processed by the microprocessor 48 which has access to the medical state parameters 144 in the state parameter memory 142. The C++ object 49 is termed in this disclosure "VSParameter" and utilises a library provided by Google termed "protocol buffers" as well as a signal mechanism which is based on the Nokia Qt-frame work. Objects in the VSParameter object 49 include data such as name of the medical state parameter, values of the medical state parameter, creator identification, last modification identification, date of last modification, etc.

One of the VSParameter objects is termed "Heart Rate Parameter" and is the medical state parameter that the instructor uses to change the heart rate of the manikin 20. The movement of the sliding bar 530 is detected by the touchscreen display and the value of the heart rate parameter is changed by looking at the initial value of the heart rate parameter and the length of the slide carried out by the finger (or the stylus) on the sliding bar 530. It will be seen that a digital display of the new parameter value is generated and shown the display 46.

One of the further objects in the C++ object is the "Parameter Controller" 49a. The function of the parameter controller 49a is to inform the base unit 30 of the change of parameter values in one or more of the VSParameter objects. The change of the heart rate parameter discussed above will be monitored by the parameter controller 49a and the base unit 30 will be informed of the change as discussed below.

The subscreen shown in Fig. 4B also illustrates the manner in which the ECG can be changed. Currently this is set to be a regular ("sinus") form as shown in area 540. However, selecting the panel 54 underneath will allow the form of the ECG to be changed which will then be displayed in the area 540.

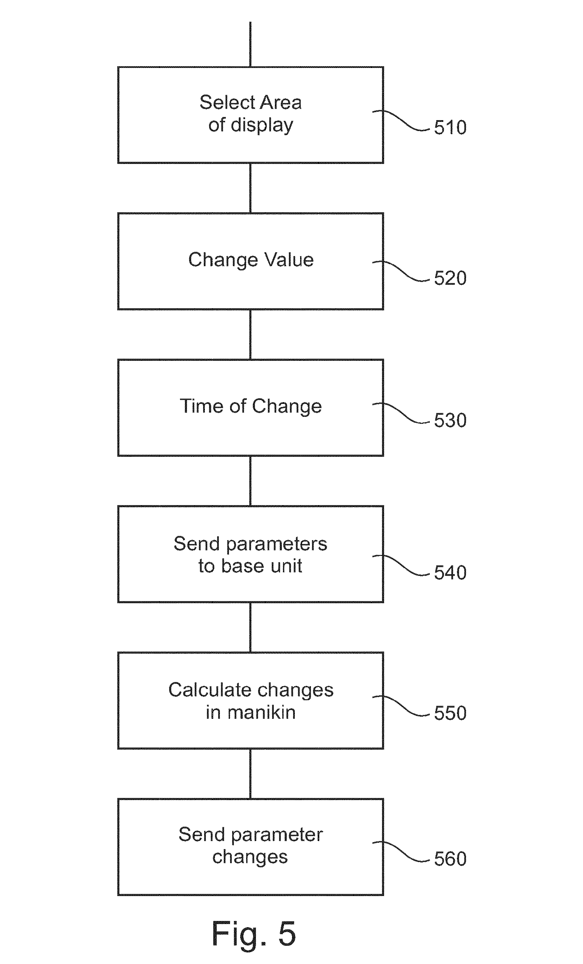

A typical example will serve to illustrate this operation and is shown in Fig. 5. Let us suppose for example that the heart rate is 80 beats per minute, as seen from Fig. 4b, and that the instructor wishes to change this value to 40 beats per minute to represent a weakening patient. The instructor can select the appropriate area of the display 46 for changing the heart rate in step 510. The sliding bar 530 for the heart rate is displayed in step 520 and has a sliding pointer 535. The instructor can move the value from 80 beats per minute to 40 beats per minute using his or her finger on the sliding pointer 535. In an alternative aspect of the invention the new value for the heart rate could be entered using a virtual keyboard. The value of the heart rate parameter in the VSParameter object 49 will be changed

The instructor enters in step 530 the time over which the change from 80 beats per minute to 40 beats per minute should take place. This is done by using the subscreen shown in Fig. 4C in which the transition time is set to be 1 minute and 40 seconds by sliding the sliding pointer 535 along the sliding bar 530 on the right hand side. This value becomes the value of the time change parameter in the VSParameter 49.

The control unit 40 will send a heart rate signal to the base unit 30 indicating the change in the heart rate parameter(i. e. the change in the value of the heart rate) in step 540, as explained below

The change of the heart rate in step 520 and the time over which the change should take place in step 530 are monitored by the parameter controller 49a. The parameter controller 49a will inform the network client 41 on the control device 40 that some of the medical state parameter objects have new value (in this case the medical state parameter objects representing the heart rate and the associated time change parameter). The network client 41 will ask for a list of all of the medical state parameter objects having a new value and request serialisation of the medical state parameter objects with a protocol buffer in the control device 40.

The network client will then send the serialised parameter objects as a serialised data stream 25a to the base unit 30 over the data link 25. The base unit 30 will receive the medical state parameter objects and will create new parameter objects in the base unit memory 38 based on the serialised data stream 29. The base unit network server 27 will attempt to register the new parameter objects in a parameter base unit application 28.

If the base unit network server 27 observes that one or more of the parameter objects has an identical name with an existing one of the parameter objects in the base unit memory 38, than the value of the existing one of the parameter objects in the base unit memory 38 will receive the new value in step 540. If the base unit network server 27 establishes that none of the parameter objects in the base unit memory 38 exists with this name, than a new parameter object will be established in the base unit memory 38 and will be available for application.

This new value may be synchronised with any other clients. In the example given in Fig. 5, The heart rate object in the base unit 30 will record that the hear rate object has a new value and will send a signal to any other parameter objects that subscribes to changes in the value of the heart rate object and notify these other objects that the value of the parameter of the heart rate has been changed in the base unit 30.

In step 540 the base unit 30 stores the change of values in the base unit memory 38 and calculates in step 550 using the base unit processor 39 the change in parameters that needs to be sent to the manikin 20. The base unit processor 39 will not only change the parameters for the actuators 220 relating to the pulses (which are sent across the data link 25 through the cable connector 32, as explained above). The base unit processor 39 will also calculate other changes in parameters that may be necessary. For example the base unit processor 39 may send to the CPR meter 70 changes in the heart beat and adapt the display on the CPR meter 70 to take into account the change in heart beat. These changes are sent in step 560. The instructor does not have to think about doing these consequential changes. The instructor can continue to observe the learner. The base unit 30 will use the time change parameter received from the control unit 40 (and shown on Fig. 4c) to calculate the time over which the value of the hear rate in the manikin 20 changes.

The base unit processor 39 will calculate the changes over time so that the changes in the necessary parameters are sent over the time specified by the instructor for the change to take place. In other words, the base unit processor 39 sends in step 560 a substantially continuous set of changes in parameters until the change is completed.

It is also possible to select a subscreen to change the blood pressure and set the pulse strength which is shown in Fig 4D. This shows three sliding bars 530a, 530b, 530c for changing the blood pressure and pulse rates.

The sliding bar 530a is identical with the sliding bar 530for changing the value of the heart rate parameter in Fig. 4b. The sliding bar 530b is for changing the value of the blood pressure and the sliding bar 530c is for changing the value of the isotonic blood pressure. It will be seen that the two values are shown in a digital manner in the top left hand corner of the display 46. In a pulse strength sub-display, the pulse is shown as being of normal strength. The instructor could vary the value of the pulse strength and also the positions in which the pulse is detectable by changing the icon in the pulse strength sub-display 550.

The effect of changing the parameters is similar to the method described in Fig. 5 and in connection with Figs. 4b and 4c. The values of the parameter objects representing the blood pressure and the pulse will be changed in the control device 40. The parameter controller will inform the network client that these parameter objects have a new value and, as described above the network client will than ask for a list of parameter objects having a new value and request serialisation with the protocol buffer.

The parameter objects will be send to the base unit 30 via the serialised data stream 25a and will than be stored in the equivalent parameter objects in the base unit memory 39.

In this case the base unit 30 will cause the actuators 220 which represent the pulse to simulate the pulse rate of the manikin 20 by sending control signals over the appropriate signal lines in the control and data leads 60. The base unit 30 can adjust the air pressure at the air pressure input 330 connected to the blood pressure cuff 22 so that the learner can use the blood pressure cuff 22 to check the simulated blood pressure of the manikin 20.

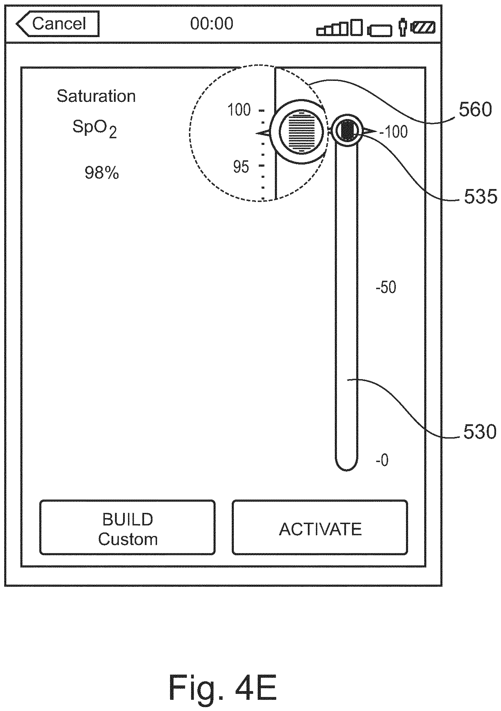

A further aspect of the display 46 is shown with respect to Fig. 4e. Fig. 4e shows an example of one of the medical state parameters relating to the saturation of blood with oxygen. It will be seen that the sliding barbath 530 goes from 0 % to 100 %. Fig. 4e includes a virtual "magnifying glass" 560 which can be activated by the instructor keeping a finger on the slider 535 of the display screen 46. It will be seen that the magnifying glass 560 shows a scale of the sliding bar 530 in much more detail and enables the instructor to accurately set a value of the medical state parameter represented by the sliding bar 530. In the aspect shown in Fig. 4e the instructor is able to accurately set the saturation level of the blood to a value of 98 %, by moving the sliding pointer.

| Reference Numeral | Description |
|---|---|
| | |
| 10 | Educational Apparatus |
| 20 | Patient Simulator / Manikin |
| 21 | Thermometer |
| 22 | Blood pressure cuff |
| 23 | Manikin speakers |
| 24 | Manikin audio input |
| 25 | Data link |
| 25a | Serialised data stream |
| 26 | Embedded processors |
| 27 | Base unit network server |
| 28 | Parameter base unit application |
| 29 | Controller area network interface |
| 30 | Base Unit |
| 31 | Base unit battery |
| 32 | Cable connector |
| 33 | Air pressure input |
| 34 | Audio output |
| 35 | Audio input |
| 36 | Base unit headset |
| 37 | Sounds generator |
| 38 | Base unit memory |
| 39 | Base unit processor |
| 40 | Control device |
| 41 | Network client |
| 42 | Control device battery |
| 44 | Memory |
| 45 | Buffer |
| 46 | Display |
| 48 | Micro processor |
| 49 | C++ object |
| 49a | Parameter Controller |
| 50 | Audio input |
| 52 | Audio output |
| 54 | Accelerometer |
| 56 | Data interface |
| 58 | Control device headset |
| 60 | Control and data Leads |
| 70 | CPR meter |
| 75 | CPR meter connection |
| 110 | Display |
| 112 | Location |
| 114 | Magnified Image |
| 120 | Input |
| 130 | Graphics Processor |
| 132 | GUI Application |
| 142 | State Parameter Memory |
| 144 | Medical State Parameters |
| 150 | Communications Interface |
| 160 | Accelerometer |
| 170 | View |
| 210 | Loudspeaker |
| 215 | Microphones |
| 220 | Actuators |
| 225 | Sensors |
| 230 | Skin |
| 240 | Clothing |
| 250 | Head |
| 251 | Face |
| 252 | Ear |
| 253 | Nose |
| 254 | Eye |
| 255 | Mouth |
| 256 | Hand |
| 257 | Wrist |
| 258 | Neck |
| 259 | Knee |
| 260 | Torso |
| 261 | Thigh |
| 262 | Chest |
| 263 | Lung |
| 264 | Heart |
| 265 | Elbow |
| 270 | Manikin Identification |
| 300 | Audio connections |
| 310 | Instructor talk button |
| 320 | Router |
| 330 | Manikin vocal enable switch |
| 340 | Headset talk switch |
| 350 | Instructor enable switch |
| 360 | Recording device |
| 365 | Memory switch |
| 410 | Graphical user interface application |
| 500 | Subscreen selection buttoms |
| 510 | State buttons |
| 520 | Summary display area |
| 530 | Sliding bar |
| 535 | Sliding pointer |
| 540 | Area |
| 541 | Panel |
| 550 | Pulse strength sub-display |
| 560 | Magnifying glass |

## Claims

1. A computerized educational apparatus (10) for simulating medical events comprising:
- at least one patient simulator (20) having a plurality of sensors (225) and a plurality of actuators (220);
- a control device (40) located remotely from the patient simulator (20) comprising a state parameter memory (142) for storing a plurality of medical state parameter coupled to said control processor (140), wherein the plurality of medical state parameters are representative of a medical state of at least one of the at least one patient simulators (20); and;
- a base unit (30) in data contact with the patient simulator (20) and the control device (40); and
- one or more medical parameter measurement devices (21, 22, 70) in data contact with the base unit (30), wherein
- the control device (40) is adapted to communicate with the base unit (30) of the at least one patient simulator (10) for the transfer of medical state parameter values representative of the medical state parameters (144) to the base unit (30) of the at least one patient simulator (20); and
- the base unit (30) is adapted to store changes received from the control device (40) in a base unit memory (38) and is further adapted to calculate in a base unit processor (39) changes that need to be send to the patient simulator (20).

2. The computerised educational apparatus (10) of claim 1, wherein the base unit processor (39) is adapted to take into account time change parameter received from the control device (40) for calculating changes over time and sending continuously changes in parameters to the patient simulator (20) until the change is completed.

3. The computerised educational apparatus (10) of claim 1 or 2, wherein the one or more medical parameter measurement devices comprise at least one of a CPR meter (70), a thermometer (21), and a blood pressure cuff (22).

4. A control device (40) for controlling at least one patent simulator (20) with a plurality of sensors (225) and a plurality of actuators (220), wherein
- the control device (40) is located remotely from the patient simulator (20);
- the control device (40) is adapted in use to be in data contact with a base unit (30), wherein the base unit is in use data contactable with the patient simulator (20) and with one or more medical parameter measurement devices (21, 22, 70)
- the control device comprises a state parameter memory (142) for storing a plurality of medical state parameter coupled to a control processor (140), wherein the plurality of medical state parameters are representative of a medical state of at least one of the at least one patient simulators (20); and
- the control device further comprises a communications interface (150) adapted to communicate with the base unit (30) of the at least one patient simulator (10) for the transfer of medical state parameter values representative of the medical state parameters (144) to the base unit (30) at least one patient simulator (20).

5. The control device (40) of claim 4 for controlling the at least one patient simulator (20), comprising:
- a display (110);
- an input device (120);
- a graphics processor (130) for producing the display.

6. The control device (40) of claim 4 or 5, further comprising an accelerometer (160) connected to the graphics processor (130), the accelerometer (160) detecting the position of the portable control device (40) and cooperating with the graphics processor (130) to adapt a view (46) on the display (110).

7. The control device (40) of any of claims 4 to 6, wherein a first actuation of the input device (120) at a location (112) is adapted to cooperate with the graphics process (130) to produce at least a partially magnified image (114) on the display (110).

8. The control device (40) of any one of claims 4 to 7, wherein a second actuation of the input device (120) at a location (112) is adapted to change the values of at least one of the medical state parameters (144).

9. The control device (40) of any one of claims 4 to 8, wherein the communication interface (150) comprises a medical state parameter buffer for storing the medical state parameter s(144).

10. The control device (40) of any one of claims 4 to 9 further comprising a medical state parameter controller for monitoring changes of the medical state parameter values.

11. A patient simulator (20) comprising:
- at least one loudspeaker (210);
- at least one or more body parts (250, 260)
- at least one or more actuators (220) adapted to move at least one or more of the body parts (250, 260);
- at least one or more sensors (225) adapted to sense manipulation of at least one of the one or more body parts (250, 260); and
- at least one or more embedded controllers (26) adapted to transceive data signals from a base unit (30) and being connected to at least one of the one or more sensors (225) or the one or more actuators (220),
- wherein the patient simulator is adapted to be in data contact with a base unit (30) located remotely from the patient simulator (20) and wherein the base unit (30) is adapted to store changes received from a control device (40) in a base unit memory (38) and is further adapted to calculate in a base unit processor (39) changes that need to be send to the patient simulator (20).

12. The patient simulator (20) of claim 11, further comprising control and data leads (60) for transceiving of the data signals.

13. The patient simulator (20) of claim 11 or 12, wherein the data signals comprise signals representative of medical state parameter values.

14. The patient simulator (20) of any one of claims 11 to 13, further comprising a manikin identification (270).

15. A method for simulating patient care in a patient simulator (20) comprising:
- setting one or more medical state parameters in a control device (40);
- detecting parameter changes in at least one of the one ore more medical state parameters in the control device (40);
- transmitting said parameter changes from the control device(40) to a base unit (3 0);
- storing changes received from the control device (40) in a base unit (30) and calculating in a base unit (30) changes that need to be adjusted in the patient simulator (20) and
- adjusting at least one of a plurality of actuators (220) or a plurality of sensors (225) in the patient simulator (20).

16. The method of claim 15 further comprising
- transmitting at least one of said parameter changes to a medical parameter measurement devices.

17. The method of claim 15 or 16 further comprising:
- selecting in a control device (40) a medical state parameter from a plurality of medical state parameters;
- displaying first medical state parameter value of the selected medical state parameter in the control device (40);
- detecting movement of an object from a first position corresponding to the first medical state parameter value to a second position corresponding to a second medical state parameter value of the selected medical state parameter; and
- storing the second medical state parameter value.

18. The method of one of claims 15 to 17 further comprising:
- transmitting the second medical state parameter to a patient simulator (20)

19. The method of one of claims 15 or 18, further comprising:
- selecting a time parameter value; and
- transmitting the time parameter value to the patient simulator (20).
